# EUROPEAN PATENT SPECIFICATION

(11) **EP 1 928 451 B1**
(45) Date of publication and mention of the grant of the patent: **28.12.2011**
(21) Application number: 05775692.6
(22) Date of filing: 26.07.2005
(51) Int. Cl.: A61K 31/325

(54) **METHODS FOR TREATING SUBSTANCE-RELATED DISORDERS**
VERFAHREN ZUR BEHANDLUNG VON SUBSTANZBEDINGTEN ERKRANKUNGEN
PROCEDES POUR TRAITER DES TROUBLES LIES A DES SUBSTANCES

(43) Date of publication of application: 11.06.2008
(73) Proprietor: SK Biopharmaceuticals Co., LTD, Jongro-gu Seoul 110-110 (KR)
(72) Inventor: PLATA-SALAMAN, Carlos, R., Ambler, Pennsylvania 19002 (US); ZHAO, Boyu, Lansdale, Pennsylvania 19446 (US); TWYMAN, Roy, E., Doylestown, Pennsylvania 18901 (US)
(74) Representative: Warner, James Alexander
(86) International application number: PCT/US2005/026439
(87) International publication number: WO 2007/018496

(56) References cited:
- WO-A-02/067926
- WO-A-02/067927
- US-A- 3 313 692
- US-A1- 2004 171 679

## Description

### FIELD OF THE INVENTION

This invention is directed to methods for preventing, treating or ameliorating Substance-Related Disorders. More particularly, this invention is directed to the use of certain halogenated 2-phenyl-1, 2-ethanediol monocarbamate or dicarbamate compounds, either alone or in combination with other medications, for preventing, treating or ameliorating Substance-Related Disorders, including Substance Dependence, Abuse and Substance - Induced Disorders.

### BACKGROUND OF THE INVENTION

Substance-Related Disorders include disorders related to the taking of a drug of abuse and include Substance Use Disorders such as Substance Dependence and Abuse and Substance-Induced Disorders such as Substance Withdrawal syndromes and Substance Induced Psychosis and Mood Disorders. Substance Dependence is a cluster of cognitive, behavioral and physiological symptoms the essential feature of which is that the individual affected continues the use of the substance despite significant substance-related problems.

Many drugs can cause dependence or physical and/or psychological addiction. Opiates (such as heroin, opium, morphine and the like), sympathomimetics (such as cocaine, amphetamines, methamphetamines and the like), sedative-hypnotics (such as ethanol, benzodiazepines, barbiturates, inhalants, phencyclidines and the like) and those with a combination of opioid and sympathomimetic effects (such as nicotine and the like) are considered to be drugs that cause dependence or physical and/or psychological addiction.

Substance Dependence or addiction is characterized by a craving or compulsion for taking the substance and an inability to limit its intake. Additionally, drug dependence is associated with drug tolerance, the loss of effect of the drug following repeated administration, and withdrawal, the appearance of physical and behavioral symptoms when the drug is not consumed. Sensitization occurs if repeated administration of a drug leads to an increased response to each dose. Tolerance, sensitization and withdrawal are phenomena evidencing a change in the central nervous system resulting from continued use of drugs that cause addiction. Such changes motivate the addicted individual to continue consuming the drug despite serious social, legal, physical and/or professional consequences.

Direct, indirect or trans-synaptic changes to dopamine release and/or reuptake have been implicated in disorders resulting from addictive behaviors and in rewarding and/or reinforcing drug use, as in a "Reward Deficiency Syndrome." Dopamine is a monoamine neurotransmitter that physiologically functions in the forebrain's integration of information in sensory, limbic and motor systems. Since reduced dopaminergic functions have been found in individuals with a minor allele of the dopamine receptor, the dopamine receptor appears to be a reinforcement or reward gene. Variants of the dopamine receptor gene have been associated with alcoholism, obesity, pathological gambling, attention deficit hyperactivity disorder, Tourette syndrome, cocaine dependence, nicotine dependence, polysubstance abuse and other drug dependencies.

Attempts to treat Substance Dependence have included pharmaceutical and behavioral intervention. Treatment has included administering pharmacologic agents (e.g. bupropion, naltrexone, acamprosate and the like) either alone or with other agents to variously block dopamine reuptake or enhance dopamine release. Drug treatment has also been optionally coextensive with behavior modification therapies to treat addiction. (See, Hoffman et al., Front. Neuroendocrinol., 1998, 19(3):187-231; Hitri et al., Clin. Pharmacol., 1994, 17:1-22; Noble, Alcohol Supp., 1994, 2:35-43; Blum et al., Pharmacogenetics, 1995, 5:121-141; US Pat. 5, 039,680, US Pat. 5,075,341, US Pat. 5,232,934, US Pat. 5,556,837, US Pat. 5,556,838, US Pat. 5,574,052, US Pat. 5,762,925, US Pat. 6,593,367, US Pat. 6,109,269, US Pat. 6,716,868, US Pat. App. 20030144271, PCT App. WO0141763) .

Substance Abuse is a maladaptive pattern of substance use manifested by recurrent and significant adverse consequences related to the repeated use of the substance. These problems occur repeatedly for at least a 12 month period.

Substance-Induced Disorders refers to a wide variety of temporary or more chronic syndromes due to the physiological effects of the substance on the central nervous system. Such disorders may develop during or shortly after use of the substance. These include; acute intoxication and withdrawal, and a variety of substance-induced mental disorders such as; delirium, persisting dementia, persisting amnestic disorder, psychotic disorder, mood disorder, anxiety disorder, sexual dysfunction and sleep disorder.

See, Diagnostic And Statistical Manual of Mental Disorders, Fourth Edition, (DSM-IV) Pages 175-272, Published by The American Psychiatric Association, Washington, DC (1994).

There remains a need for effective pharmacologic therapies to prevent, treat or ameliorate Substance-Related Disorders resulting from the different classes of abusable drugs. In particular, there is a need for small molecule compounds that may be readily synthesized, that are potent and stabilizing mediators and restorative agents for neurotransmitter pathways and are useful for preventing, treating or ameliorating Substance-Related Disorders including Substance Use Disorders such as Substance Dependence (drug addiction and Substance Abuse and Substance-Induced Disorders, including but not limited to; acute intoxication and withdrawal, and substance-induced mental disorders such as; delirium, persisting dementia, persisting amnestic disorder, psychotic disorder, mood disorder, anxiety disorder, sexual dysfunction and sleep disorder.

Carbamate compounds have been disclosed in WO02/067927 for the treatment of psychotic disorders, in US2004/171679 for the treatment of neurodegenerative disorders in US3313692 for the treatment of the nervous system and in WO02/067926 for the treatment of movement disorders.

Substituted phenyl alkyl carbamate compounds have been described in US Pat. 3,265,728 to Bossinger, et al as useful in treating the central nervous system, having tranquilization, sedation and muscle relaxation properties of the formula: 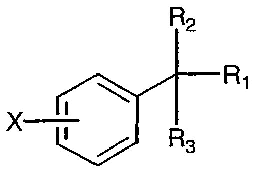 wherein R₁ is either carbamate or alkyl carbamate containing from 1 to 3 carbon atoms in the alkyl group; R₂ is either hydrogen, hydroxy, alkyl or hydroxy alkyl containing from 1 to 2 carbons; R₃ is either hydrogen or alkyl containing from 1 to 2 carbons; and X can be halogen, methyl, methoxy, phenyl, nitro or amino.

A method for inducing calming and muscle relaxation with carbamates has been described in US Pat. 3,313,692 to Bossinger, et al by administering a compound of the formula: 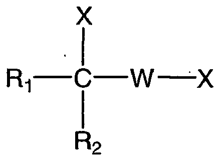 in which W represents an aliphatic radical containing less than 4 carbon atoms, wherein R₁ represents an aromatic radical, R₂ represents hydrogen or an alkyl radical containing less than 4 carbon atoms, and X represents a hydrogen, hydroxy, alkoxy or alkyl radical containing less than 4 carbon atoms or a radical of the formula: 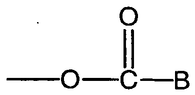 in which B represents an organic heterocyclic, ureido or hydrazino amine radical or the radical -N(R₃)₂, wherein R₃ represents hydrogen or an alkyl radical containing less than 4 carbon atoms.

Optically pure forms of substituted phenyl alkyl carbamate compounds have been described in US Pat. 6,103,759 to Choi, et al as effective for treating and preventing central nervous system disorders including convulsions, epilepsy, stroke and muscle spasm and as useful in the treatment of central nervous system diseases, particularly as anticonvulsants, antiepileptics, neuroprotective agents and centrally acting muscle relaxants and, in particular, as halogen substituted 2-phenyl-1,2-ethanediol monocarbamate and dicarbamate compounds of the formulae: 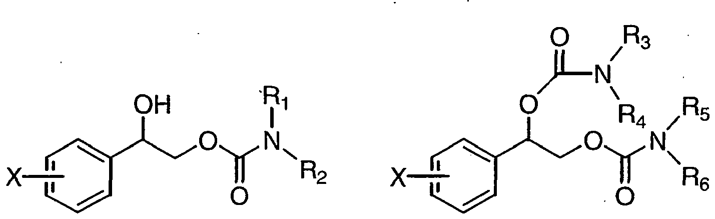 wherein one enantiomer predominates and wherein the phenyl ring is substituted at X with one to five halogen atoms selected from fluorine, chlorine, bromine or iodine atoms and R₁, R₂ , R₃, R₄, R₅ and R₆ are each selected from hydrogen and straight or branched alkyl groups with one to four carbons optionally substituted with a phenyl group with substituents selected from the group consisting of hydrogen, halogen, alkyl, alkyloxy, amino, nitro and cyano. Pure enantiomeric forms and enantiomeric mixtures are described wherein one of the enantiomers predominates in the mixture for the compounds represented by the formulae above; preferably, one of the enantiomers predominates to the extent of about 90% or greater; and, most preferably, about 98% or greater.

Recent preclinical studies have revealed previously unrecognized pharmacological properties which suggest that a form of a substituted phenyl alkyl carbamate compound is useful in preventing, treating or ameliorating a wide variety of substance-related disorders. Such monocarbamate and dicarbamate compounds thereof have not been previously described as useful for preventing, treating or ameliorating substance-related disorders such as substance dependence and the many Substance-Induced Disorders

### SUMMARY OF THE INVENTION

The present invention is directed to a carbamate compound or enantiomer selected from: 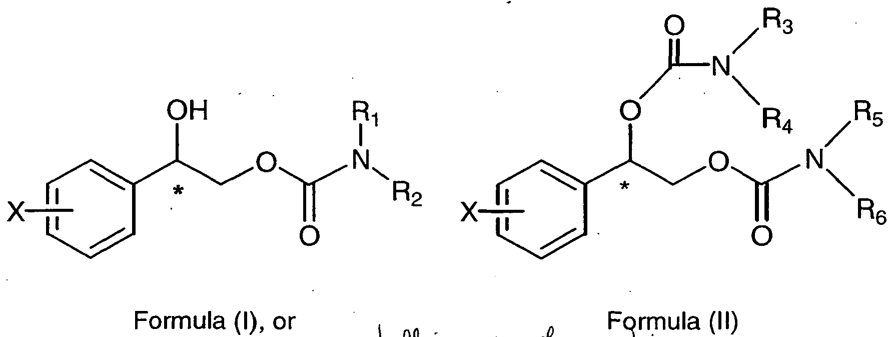 or pharmaceutically acceptable crystalline or polymorphic forms thereof, wherein all variables are as described herein for in use in preventing, treating or ameliorating substance-related disorders as claimed in claim 1

The present invention is also directed to a method for use of a compound of Formula (I) or Formula (II) for preventing, treating or ameliorating substance-induced disorders or diseases and disorders as claimed in claim 1 resulting from the use of dependence inducing or addictive drugs.

The method of the present invention includes the use of a compound of Formula (I) or Formula (II) for modulating the effect of substance dependence or addiction associated diseases and disorders and symptoms thereof or the effect of physiologic events associated with such diseases and disorders, i.e., substance-induced disorders, as claimed in claim 1.

### DETAILED DESCRIPTION OF THE INVENTION

The present invention describes a method for preventing, treating or ameliorating Substance-Related Disorders in a subject in need thereof comprising administering to the subject an effective amount of a compound selected from Formula (I) or Formula (II): 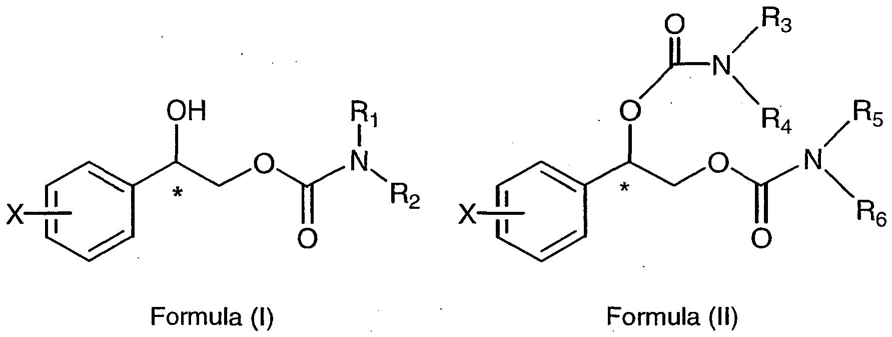 or pharmaceutically acceptable forms thereof, wherein
phenyl is substituted at X with one to five halogen atoms selected from the group consisting of fluorine, chlorine, bromine and iodine, and
R₁, R₂, R₃, R₄, R₅ and R₆ are independently selected from the group consisting of hydrogen and C₁-C₄ alkyl, wherein C₁-C₄ alkyl is optionally substituted with phenyl (and, wherein phenyl is optionally substituted with substituents independently selected from the group consisting of halogen, C₁-C₄ alkyl, C₁-C₄ alkoxy, amino, nitro and cyano).

The present invention describes a method for preventing, treating or ameliorating substance-related disorders including substance dependence or drug addiction in a subject in need thereof comprising administering to the subject an effective amount of a pharmaceutical composition comprising a pharmaceutically acceptable carrier and a compound selected from Formula (I) or Formula (II).

The present invention describes methods for preventing, treating or ameliorating substance-induced disorders or diseases and disorders resulting from the use of addictive drugs and for modulating the effect of addiction associated diseases and disorders and symptoms thereof or the effect of physiologic events associated with such diseases and disorders in a subject in need thereof comprising administering to the subject an effective amount of a compound selected from Formula (I) or Formula (II) or administering to the subject an effective amount of a pharmaceutical composition comprising a pharmaceutically acceptable carrier and a compound selected from Formula (I) or Formula (II).

The present invention describes methods for the preventing, treating or ameliorating substance-induced disorders including substance dependence comprising administering to a subject, in need of such treatment, an effective amount of a combination product having a compound selected from Formula (I) or Formula (II) or a pharmaceutical composition comprising a pharmaceutically acceptable carrier and a compound selected from Formula (I) or Formula (II) in combination with an effective amount of one or more compounds known to treat substance-related disorders. Compounds known to treat substance-related disorders include, but are not limited to; naltrexone, naloxone or other opioid receptor antagonists, fluoxetine or other SSRI antidepressants, thyrotropin-releasing hormone analogues, venlafaxine or other SNRI antidepressants, MAO Inhibitors antidepressants, tricyclic antidepressants, bupropion, lithium carbonate, anticonvulsants, acamprosate, disulfiram (antabuse), calcium channel blockers, serotonin antagonists, GABA-altering drugs, dopaminergic drugs, an N-methyl-D-aspartic acid (NMDA) glutamate receptor modulator and the like.

The methods of the present invention further describe preventing, treating or ameliorating substance-induced disorders in a subject in need thereof comprising administering an effective amount of a compound of the present invention and a pharmaceutical composition or combination product thereof in a therapy regimen coextensive with behavior modification.

This invention includes the use of a compound selected from Formula (I) or Formula (II) or pharmaceutical composition thereof for the preparation of a medicament for preventing, treating or ameliorating substance-related disorders such as substance dependence and abuse and substance induced disorders.

Examples of a compound selected from Formula (I) for use in the present method include an enantiomer of Formula (I) or an enantiomer of Formula (I) in an enantiomeric mixture wherein one enantiomer predominates.

Examples of a compound selected from Formula (II) for use in the present method include an enantiomer of Formula (II) or an enantiomer of Formula (II) in an enantiomeric mixture wherein one enantiomer predominates.

For an enantiomeric mixture of Formula (I) or Formula (II) wherein one enantiomer predominates, the enantiomer predominates to the extent of about 90% or greater. Examples of the present invention also include enantiomeric mixtures wherein said enantiomer predominates to the extent of about 98% or greater.

Other examples of said compound of Formula (I) or Formula (II) include compounds wherein X is chlorine, wherein X is substituted at the ortho position of the phenyl ring of Formula (I) or Formula (II) and, wherein R₁, R₂, R₃, R₄, R₅ and R₆ are hydrogen.

An example of the present method includes the use of an enantiomer selected from Formula (I) or Formula (II) or an enantiomeric mixture thereof wherein one enantiomer predominates, wherein X is chlorine and, wherein X is substituted at the ortho position of the phenyl ring.

The present method also includes the use of an enantiomer selected from Formula (I) or Formula (II) or an enantiomeric mixture thereof wherein one enantiomer predominates and, wherein R₁, R₂, R₃, R₄, R₅ and R₆ are hydrogen.

For enantiomeric mixtures wherein one enantiomer selected from Formula (I) or Formula (II) predominates, said enantiomer predominates to the extent of about 90% or greater. Enantiomeric mixtures within the scope of the present invention also include those wherein said enantiomer predominates to the extent of about 98% or greater.

The present invention describes a method for preventing, treating or ameliorating substance related disorders in a subject in need thereof comprising administering to the subject an effective amount of an enantiomer selected from Formula (Ia) or Formula (IIa) or an enantiomeric mixture of Formula (Ia) or Formula (IIa) wherein one enantiomer predominates: 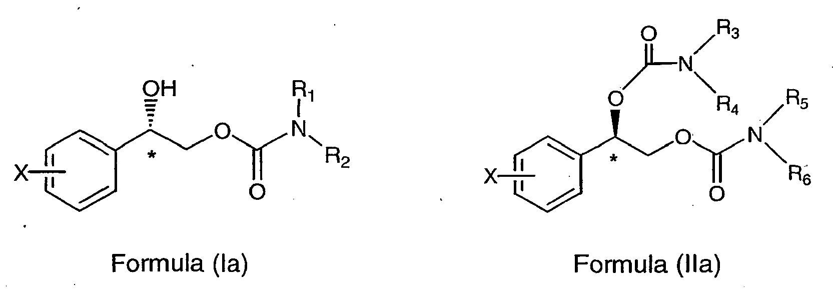 or pharmaceutically acceptable forms thereof, wherein
phenyl is substituted at X. with one to five halogen atoms selected from the group consisting of fluorine, chlorine, bromine and iodine, and
R₁, R₂, R₃, R₄, R₅ and R₆ are independently selected from the group consisting of hydrogen and C₁-C₄ alkyl, wherein C₁-C₄ alkyl is optionally substituted with phenyl (and, wherein phenyl is optionally substituted with substituents independently selected from the group consisting of halogen, C₁-C₄ alkyl, C₁-C₄ alkoxy, amino, nitro and cyano).

Examples of the present method include the use of an enantiomer selected from Formula (Ia) or Formula (IIa) or an enantiomeric mixture thereof wherein one enantiomer predominates, wherein X is chlorine and, wherein X is substituted at the ortho position of the phenyl ring.

The present method also includes the use of an enantiomer selected from Formula (Ia) or Formula (IIa) or enantiomeric mixture thereof wherein one enantiomer predominates and, wherein R₁, R₂, R₃, R₄, R₅ and R₆ are hydrogen.

For enantiomeric mixtures wherein one enantiomer selected from Formula (Ia) or Formula (IIa) predominates, said enantiomer predominates to the extent of about 90% or greater. Enantiomeric mixtures within the scope of the present invention include those wherein said enantiomer predominates to the extent of about 98% or greater.

The present invention describes a method for preventing, treating or ameliorating substance related disorders in a subject in need thereof comprising administering to the subject an effective amount of an enantiomer selected from Formula (Ib) or Formula (IIb) or an enantiomeric mixture of Formula (Ib) or Formula (IIb) wherein one enantiomer predominates:

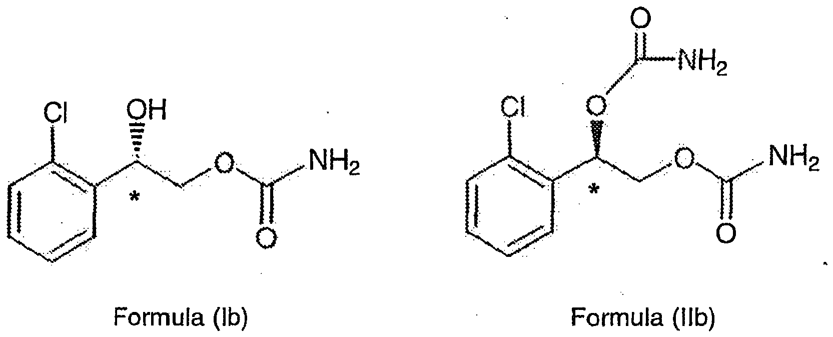

For enantiomeric mixtures wherein one enantiomer selected from Formula (Ib) or Formula (IIb) predominates, said enantiomer predominates to the extent of about 90% or greater. Enantiomeric mixtures within the scope of the present invention include those wherein said enantiomer predominates to the extent of about 98% or greater.

The present invention is directed to compounds of Formula (I) or Formula (II) or pharmaceutically acceptable crystalline or polymorphic forms thereof.

The term "isomer" refers to compounds that have the same composition and molecular weight but differ in physical and/or chemical properties. Such substances have the same number and kind of atoms but differ in structure. The structural difference may be in constitution (geometric isomers) or in an ability to rotate the plane of polarized light (stereoisomers). The term "stereoisomer" refers to isomers of identical constitution that differ in the arrangement of their atoms in space. Enantiomers and diastereomers are stereoisomers wherein an asymmetrically substituted carbon atom acts as a chiral center. The term "chiral" refers to a molecule that is not superposable on its mirror image, implying the absence of an axis and a plane or center of symmetry.

It is apparent to those skilled in the art that the compounds of the invention are present as racemates, enantiomers and enantiomeric mixtures thereof. A carbamate enantiomer selected from Formula (I), Formula (II), Formula (Ia), Formula (IIa), Formula (Ib) or Formula (IIb) contains an asymmetric chiral carbon atom at the benzylic position, which is the aliphatic carbon adjacent to the phenyl ring (represented by the asterisk in the structural formulae).

The term "racemate" or "racemic mixture" refers to a compound of equimolar quantities of two enantiomeric species, wherein the compound is devoid of optical activity. The term "optical activity" refers to the degree to which a chiral molecule or nonracemic mixture of chiral molecules rotates the plane of polarized light.

The term "enantiomer" refers to one of a pair of molecular species that are mirror images of each other and are not superposable. The term "diastereomer" refers to stereoisomers that are not related as mirror images. The symbols "R" and "S" represent the configuration of substituents around a chiral carbon atom(s). The symbols "R*" and "S*" denote the relative configurations of of substituents around a chiral carbon atom(s)..

The isomeric descriptors "R," "S," "S*" or "R*" are used as described herein for indicating atom configuration(s) relative to a core molecule and are intended to be used as defined in the literature (IUPAC Recommendations for Fundamental Stereochemistry (Section E), Pure Appl. Chem., 1976, 45:13-30)(incorporated by reference herein).

Compounds of the present invention may be prepared as described in Bossinger '728, Bossinger '692 and Choi '759 . It is understood that substituents and substitution patterns on the compounds of this invention can be selected by one of ordinary skill in the art to provide compounds that are chemically stable and that can be readily synthesized by techniques known in the art as well as those methods set forth herein.

The present invention provides a method for preventing, treating or ameliorating substance dependence in a subject in need thereof comprising administering to the subject an effective amount of a compound selected from Formula (I) or Formula (II).

The present invention also provides a method for use of a compound of Formula (I) or Formula (II) in preventing, treating or ameliorating substance induced disorders.

The method of the present invention includes the use of said compound for modulating the effect of substance dependence associated diseases and disorders and symptoms thereof or the effect of physiologic events associated with such diseases and disorders.

The methods of the present invention further include the use of said compound, wherein said compound is an enantiomer of a compound selected from Formula (I) or Formula (II); or, wherein said compound is an enantiomer selected from Formula (Ia), Formula (IIa), Formula (Ib) or Formula (IIb); or, wherein said compound is an enantiomer of a compound selected from Formula (I) or Formula (II) in an enantiomeric mixture wherein said enantiomer predominates; or, wherein said compound is an enantiomer selected from Formula (Ia), Formula (IIa), Formula (Ib) or Formula (IIb) in an enantiomeric mixture wherein said enantiomer predominates.

The present methods also include the use of a pharmaceutical composition comprising a pharmaceutically acceptable carrier and a compound selected from Formula (I) or Formula (II) for preventing, treating or ameliorating substance related disorders including diseases and disorders resulting from the use of addictive drugs or for modulating the effect of addiction associated diseases and disorders and symptoms thereof or the effect of physiologic events associated with such diseases and disorders in a subject in need thereof in a subject in need thereof.

These methods include the use of a compound selected from Formula (I) or Formula (II) for the preparation of a medicament for preventing, treating or ameliorating substance related disorders including diseases and disorders resulting from the use of addictive drugs or for modulating the effect of addiction associated diseases and disorders and symptoms thereof or the effect of physiologic events associated with such diseases and disorders in a subject in need thereof.

In cases where compounds are sufficiently basic or acidic to form stable nontoxic acid or base salts, administration of the compounds as salts may be appropriate. Examples of pharmaceutically acceptable salts are organic acid addition salts formed with acids which form a physiological acceptable anion, for example, tosylate, methanesulfonate, acetate, citrate, malonate, tartarate, succinate, benzoate, ascorbate, alpha-ketoglutarate and alpha-glycerophosphate. Suitable inorganic salts may also be formed, including hydrochloride, sulfate, nitrate bicarbonate and carbonate salts.

Pharmaceutically acceptable salts may be obtained using standard procedures well known in the art, for example by reacting a sufficiently basic compound such as an amine with a suitable acid affording a physiologically acceptable anion. Alkali metal, for example; sodium, potassium or lithium, or alkaline earth metals, for example calcium salts of carboxylic acids can also be made.

The method of the present invention is also directed to a method for preventing, treating or ameliorating substance related disorders including drug addiction, diseases and disorders resulting from the use of addictive drugs or for modulating the effect of addiction associated diseases and disorders and symptoms thereof or the effect of physiologic events associated with such diseases and disorders in a subject in need thereof comprising administering to the subject an effective amount of a compound of Formula (I) or Formula (II), a pharmaceutical composition thereof, a pharmaceutical composition containing one or more of said compounds or a pharmaceutical composition containing one or more of said compounds and a safe and effective amount of one or more additional agents.

The term "substance dependence" also referred to as "drug addiction" refers to physical and/or psychological addiction to opiates (such as heroin, opium, morphine and the like), sympathomimetics (such as cocaine, amphetamines, methamphetamines and the like), sedative-hypnotics (such as ethanol, benzodiazepines, barbiturates, phencyclidines and the like), those with a combination of effects (such as nicotine and the like) and the like or mixtures thereof.

The phrases "substance related disorders" also referred to as "diseases and disorders resulting from the use of addictive drugs" and "addiction associated diseases and disorders and symptoms thereof" and "physiologic events associated with such diseases and disorders" refer to drug withdrawal disorders such as alcohol withdrawal (with or without perceptual disturbances and/or delirium), amphetamine withdrawal, cocaine withdrawal, nicotine withdrawal, opioid withdrawal, sedative withdrawal, hypnotic withdrawal, anxiolytic withdrawal (with or without perceptual disturbances and/or delirium), nicotine withdrawal (with or without disturbances associated with smoking cessation) and withdrawal symptoms due to other substances, substance-induced anxiety disorder with onset during withdrawal; vulnerability even after long periods of absence to environmental trigger induced craving; substance-induced mood disorder with onset during withdrawal; and substance-induced sleep disorder with onset during withdrawal and other substance-induced disorders, including but not limited to; acute intoxication and withdrawal, and substance-induced mental disorders such as; delirium, persisting dementia, persisting amnestic disorder, psychotic disorder, mood disorder, anxiety disorder, sexual dysfunction and sleep disorder.

The term "preventing, treating or ameliorating" refers to (i) preventing a disease, disorder or condition from occurring in an animal which may be predisposed to the disease or disorder and/or effects thereof but has not yet been diagnosed as having it; (ii) inhibiting or arresting the development of the disease, disorder or condition and (iii) relieving or causing regression of the disease, disorder or condition.

In relation to substance-related disorders , the term "preventing, treating or ameliorating" also refers to suppressing the psychological addiction or physical tolerance to the drug of abuse, and relieving or preventing a withdrawal syndrome resulting from the drug dependence.

The term "administering" refers to a means for preventing, treating or ameliorating substance dependence, preventing, treating or ameliorating diseases and disorders resulting from the use of addictive drugs or modulating the effect of addiction associated diseases and disorders and symptoms thereof or the effect of physiologic events associated with such diseases and disorders with an instant compound. Such means include therapeutically or prophylactically administering an effective amount of a compound, composition or medicament of the present invention at different times during the course of a therapy or concurrently in a combination form. Prophylactic administration can occur prior to the manifestation of symptoms characteristic of drug addiction such that diseases and disorders resulting from the use of addictive drugs or the effect of addiction associated diseases and disorders and symptoms thereof or the effect of physiologic events associated with such diseases and disorders is prevented, treated, ameliorated or, alternatively, delayed in its progression. The methods of the present invention are further to be understood as embracing all possible therapeutic or prophylatic treatment regimens to be imagined by those skilled in the art.

The term "subject" refers to an animal, preferably a mammal, most preferably a human, who has been the object of treatment, observation or experiment.

The term "effective amount" refers to that amount of active compound or pharmaceutical agent that elicits the biological or medicinal response in a tissue system, animal or human, that is being sough by a researcher, veterinarian, medical doctor, or other clinician, which includes therapeutic alleviation of the symptoms of the disease or disorder being treated and prophylactic.

The effective amount of a compound selected from Formula (I) or Formula (II) or pharmaceutical composition thereof may be from about 0.01 µg/Kg/dose to about 300 mg/Kg/dose. Effective amounts may also be from about 0.01 µg/Kg/dose to about 100 mg/Kg/dose. An effective amount also contemplated may be from about 0.05 µg/Kg/dose to about 10 mg/Kg/dose. Another effective amount includes from about 0.1 µg/Kg/dose to about 5 mg/Kg/dose. Therefore, the effective amount of the active ingredient contained per dosage unit (e.g., tablet, capsule, powder, injection, suppository, teaspoonful and the like) as described herein may be in a range of from about 700 ng/dose to about 21 g/dose for a subject having a weight of about 70 Kg.

The term "composition" refers to a product containing a compound of the present invention (such as a product comprising the specified ingredients in the specified amounts, as well as any product which results, directly or indirectly, from such combinations of the specified ingredients in the specified amounts).

The term "medicament" refers to a product for use in preventing, treating or ameliorating substance related disorders such as substance dependence, substance abuse or substance induced disorders in a subject in need thereof.

The term "pharmaceutically acceptable" refers to molecular entities and compositions that are of sufficient purity and quality for use in the formulation of a composition or medicament of the present invention. Since both human use (clinical and over-the-counter) and veterinary use are equally included within the scope of the present invention, a formulation would include a composition or medicament for either human or veterinary use.

The term "pharmaceutically acceptable salt thereof" refers to an acid or basic salt of the compounds of the invention that are of sufficient purity and quality for use in the formulation of a composition or medicament of the present invention and are tolerated and sufficiently non toxic to be used in a pharmaceutical preparation.

A compound selected from Formula (I) or Formula (II) or pharmaceutical composition thereof or a pharmaceutically acceptable salt thereof may be administered by any conventional route of administration including, but not limited to oral, pulmonary, intraperitoneal, intravenous, intramuscular, subcutaneous, transdermal, buccal, nasal, sublingual, ocular, rectal and vaginal. In addition, administration directly to the nervous system includes, and is not limited by present technology to, intracerebral, intraventricular, intracerebroventricular, intrathecal, intracisternal, intraspinal or peri-spinal delivery or by delivery via intracranial or intravertebral needles or catheters with or without pump devices.

It will be readily apparent to those skilled in the art that any dose or frequency of administration that provides the therapeutic or prophylactic effect described herein is suitable for use in the present invention.

Dosage regimens may be varied depending upon the requirement of the subjects (including factors associated with the particular subject being treated, including subject age, weight and diet, strength of the preparation, the advancement of the disease condition and the mode and time of administration) and the use of a particular compound of Formula (I) or Formula (II) or pharmaceutical composition thereof or a pharmaceutically acceptable salt thereof. Optimal dosages to be administered may be readily determined by those skilled in the art and will result in the need to adjust the dose to an appropriate therapeutic or prophylactic level. The use of either daily administration or post-periodic dosing may be employed.

Preferably, a compound of Formula (I) or Formula (II) or pharmaceutical composition thereof or a pharmaceutically acceptable salt thereof for preventing, treating or ameliorating substance related disorders such as substance dependence, substance abuse or substance induced disorders in a subject in need thereof is administered orally or parenterally.

In accordance with the methods of the present invention, a compound of Formula (I) or Formula (II) or pharmaceutical composition thereof described herein or a pharmaceutically acceptable salt thereof may be administered separately, at different times during the course of therapy or concurrently in divided combination or single combination forms. Advantageously, a compound selected from Formula (I) or Formula (II) or pharmaceutical compositions thereof may be administered in a single daily dose or the total daily dosage may be administered via continuous delivery or in divided doses of two, three or four times daily. The instant invention is therefore to be understood as embracing all such methods and regimes of continuous, simultaneous or alternating treatment and the term "administering" is to be interpreted accordingly.

To prepare a pharmaceutical composition of the present invention, a compound of Formula (I) or Formula (II) as the active ingredient or a pharmaceutically acceptable salt thereof is intimately admixed with a pharmaceutical carrier according to conventional pharmaceutical compounding techniques, which carrier may take a wide variety of forms depending of the form of preparation desired for administration (e.g. oral or parenteral).

Suitable pharmaceutically acceptable carriers are well known in the art. Descriptions of various pharmaceutically acceptable carriers may be found in The Handbook of Pharmaceutical Excipients, published by the American Pharmaceutical Association and the Pharmaceutical Society of Great Britain.

Methods of formulating pharmaceutical compositions have been described in numerous publications such as Pharmaceutical Dosage Forms: Tablets, Second Edition, Revised and Expanded, Volumes 1-3, edited by Lieberman et al; Pharmaceutical Dosage Forms: Parenteral Medications, Volumes 1-2, edited by Avis et al; and Pharmaceutical Dosage Forms: Disperse Systems, Volumes 1-2, edited by Lieberman et al; published by Marcel Dekker, Inc.

Preferably, a pharmaceutical composition is in a unit dosage form such as a tablet, pill, capsule, caplet, gelcap, lozenge, granule, powder, sterile parenteral solution or suspension, metered aerosol or liquid spray, drop, ampoule, autoinjector device or suppository for administration by oral, intranasal, sublingual, intraocular, transdermal, parenteral, rectal, vaginal, inhalation or insufflation means. Alternatively, the composition may be presented in a form suitable for once-weekly or once-monthly administration or may be adapted to provide a preparation for intramuscular injection.

In preparing a pharmaceutical composition having a solid dosage form for oral administration, such as a tablet, pill, capsule, caplet, gelcap, lozenge, granule or powder (each including immediate release, timed release and sustained release formulations), suitable carriers and additives include but are not limited to diluents, granulating agents, lubricants, binders, glidants, disintegrating agents and the like. If desired, tablets may be sugar coated, gelatin coated, film coated or enteric coated by standard techniques.

For preparing a solid dosage form, the principal active ingredient is mixed with a pharmaceutical carrier (e.g. conventional tableting ingredients such as diluents, binders, adhesives, disintegrants, lubricants, antiadherents and glidants). Sweeteners and flavorants may be added to chewable solid dosage forms to improve the palatability of the oral dosage form. Additionally, colorants and coatings may be added or applied to the solid dosage form for ease of identification of the drug or for aesthetic purposes. These carriers are formulated with the pharmaceutical active to provide an accurate, appropriate dose of the pharmaceutical active with a therapeutic release profile.

In preparing a pharmaceutical composition having a liquid dosage form for oral, topical and parenteral administration, any of the usual pharmaceutical media or excipients may be employed. Thus, for liquid unit dosage forms, such as suspensions (i.e. colloids, emulsions and dispersions) and solutions, suitable carriers and additives include but are not limited to pharmaceutically acceptable wetting agents, dispersants, flocculation agents, thickeners, pH control agents (i.e. buffers), osmotic agents, coloring agents, flavors, fragrances, preservatives (i.e. to control microbial growth, etc.) and a liquid vehicle may be employed. Not all of the components listed above will be required for each liquid dosage form. The liquid forms in which the novel compositions of the present invention may be incorporated for administration orally or by injection include, but are not limited to aqueous solutions, suitably flavored syrups, aqueous or oil suspensions, and flavored emulsions with edible oils such as cottonseed oil, sesame oil, coconut oil or peanut oil, as well as elixirs and similar pharmaceutical vehicles.

### Biological Experimental Example

The activities of a compound of the present invention for use in preventing, treating or ameliorating substance related disorders, diseases and disorders resulting from the use of addictive drugs or for modulating the effect of addiction associated diseases and disorders and symptoms thereof or the effect of physiologic events associated with such diseases and disorders were evaluated in the following experimental example which is intended to be a way of illustrating but not limiting the invention.

A compound of the present invention was tested in ethanol preferring rats, a model for acute and chronic drug addiction in humans using an adaptation of a published method (See, Rezvani, AH et al. Alcohol & Alcoholism, Vol. 25, No. 5, pp. 573-575, 1990, Rezvani AH, et al., Alcohol and Alcoholism (Oxford), 2000, 35(1), 76-83).

### Materials and Methods

An enantiomer of Formula (Ib) was orally administered in a dose range of from about 10 to about 90 mg/kg in test and control treatment groups of 10 rats per group over a period of 14 days. Test conditions reflected approaches for evaluating patterns of drinking (e.g., single or multiple events).

### Animals:

Adult male selectively-bred alcohol preferring (P) rats were used. The selectively-bred alcohol-preferring (P) rats have been characterized and have been widely used to study the effects of different compounds on voluntary alcohol intake (Rezvani et al, 1990, 1991, 1992a, 1992b, 1999, 2000, 2002; 2003; Murphy et al, 1988; Overstreet et al., 1992, 1999; Li and McBride, 1995).

Rats were housed individually in wire mesh cages under a constant room temperature of 22 +1 oC and 12:12 light-dark cycle (8:00-20:00, dark). Animal were fed Agway Prolab Rat/Mouse/ Hamster 3000 formula and water ad libitum.

### Protocol:

Establishment of baseline alcohol intake: Alcohol intake was determined using a standard two-bottle choice method (as described in Murphy et al. 1988; McBride et al. 1990; Rezvani et al. 1990, 1991, 1993, 1995, 1997, Rezvani and Grady, 1994).

Animals were first given free access to water in a graduated Richter tube for 1 day. After the first day, the animals were given access to only a solution of 10% (v/v) ethanol for 3 consecutive days. During this period animals became accustomed to drinking from Richter tubes and to the taste and pharmacological effects of alcohol. Thereafter, they were given free access to both water and a solution of 10% alcohol for at least 3 consecutive weeks and throughout of the period of study. The rats had free access to food. Water and alcohol intake was recorded at 6 and 24 hour after the treatment, food intake was measured at 24 hour. Animal body weight was measured every day.

### Phase I. Acute Administration:

After establishment of a stable baseline for alcohol, food, and water intake, rats were daily administered (via gavage tube) at the same time each day with either vehicle or one of the three doses (10, 30 and 90 mg/kg) of the compound using a random treatment order. The interval between treatment administration was at least 3 days. Alcohol and water intake was recorded 6 and 24 h after treatment administration and food intake was recorded at 24 hr. A total of 8-10 animals per group were used. All animals completed the acute administration study phase.

### Phase II. Chronic Administration:

The effect of chronic administration of a test compound on alcohol intake and tolerance to the anti-craving effects of the test compound was tested in naïve rats treated (via gavage tube) once a day either vehicle or test compound. A test compound dose of 45 mg/Kg was selected as an effective dose for chronic administration based on the results of the acute administration study phase. Themost effective dose was then used for the chronic adminstiration phase and administered for 14 consecutive days. Alcohol and water intake was recorded 6 and 24 h after each treatment administration and food intake was recorded at 24 hr.

### Statistical analysis of data:

The results were presented as means ±SEM (Standard Error of the Means). Alcohol intake (g/kg) was calculated by multiplying the volume of alcohol consumed in mL by 10% and 0.7893 (a factor representing ethanol density)/body weight in kg. Alcohol preference, expressed as percentage, was calculated as follows (volume of alcohol consumed (mL)/total fluid intake (mL)) x 100 (as described in Rezvani and Grady,. 1994; Rezvani et al., 1997). Statistical differences between treatment and control groups were determined by using ANOVA and Turkey Student's t test for multiple comparisons.

### Results

Table 1 provides data for the treatment groups demonstrating the effect of the test compound on acute ethanol and water intake. At a dose of about 45 mg/kg and up, the test compound had a significant effect on alcohol intake without developing a tolerance for the test compound's affect on ethanol intake.

**Table 1**

| Day | Alcohol Intake (g/Kg) | | Water Intake (g/Kg) | | Alcohol Pref (%) | | Total Fluid Intake (mL/Kg) | | Body Weight (g) | |
|---|---|---|---|---|---|---|---|---|---|---|
| | Veh | Drug | Veh | Drug | Veh | Drug | Veh | Drug | Veh | Drug |
| BL | 5.30 | 5.30 | 30 | 30 | 71 | 71 | 97 | 97 | 384 | 384 |
| 1 | 3.70 | 1.61 | 36 | 62 | 58 | 28 | 82 | 82 | 412 | 418 |
| 2 | 4.00 | 1.31 | 31 | 60 | 64 | 24 | 82 | 76 | 414 | 415 |
| 3 | 4.70 | 1.37 | 19 | 60 | 76 | 24 | 78 | 77 | 417 | 413 |
| 4 | 4.80 | 1.03 | 27 | 68 | 71 | 16 | 88 | 81 | 418 | 415 |
| 5 | 5.20 | 1.24 | 27 | 68 | 72 | 21 | 94 | 83 | 421 | 419 |
| 6 | 5.30 | 1.10 | 20 | 61 | 78 | 21 | 87 | 75 | 425 | 421 |
| 7 | 5.30 | 1.85 | 21 | 48 | 77 | 34 | 89 | 71 | 428 | 424 |
| 8 | 5.60 | 2.21 | 20 | 52 | 79 | 35 | 91 | 80 | 432 | 426 |
| 9 | 5.40 | 2.19 | 18 | 40 | 81 | 45 | 86 | 67 | 436 | 429 |
| 10 | 6.00 | 3.06 | 17 | 44 | 83 | 52 | 93 | 83 | 437 | 431 |
| 11 | 5.40 | 2.93 | 16 | 30 | 82 | 57 | 86 | 67 | 440 | 435 |
| 12 | 4.20 | 2.67 | 20 | 27 | 73 | 58 | 73 | 60 | 442 | 436 |
| 13 | 4.40 | 2.37 | 31 | 48 | 65 | 42 | 87 | 78 | 439 | 431 |
| 14 | 4.40 | 2.32 | 46 | 54 | 59 | 40 | 102 | 83 | 448 | 437 |

### References for Example Above

1. Cowen M.S., Rezvani A.H., Jarrett B., and Lawrence A.J. Ethanol consumption by Fawn-Hooded rats following abstinence: Effects of naltrexone and changes in u-upload receptors density. Alcohol: Clint. Exp. Res. 23:1008-1014, 1999.
2. Fare C.K., Rezvani A.H., Overstreet D.H., and O'Malley S. Combination pharmacotherapy in alcoholism: A novel treatment approach. CNS Spectrums 5:70-76, 2000.
3. Johnson B.A., Ait-daoud N., Bowden C.L., DiClemente, Roche J.D., Lawson K. Oral topiramate for treatment of alcohol dependence: a randomized controlled trial. The Lancet 361:1677-1685, 2003.
4. Li T.K., McBride W.J. Pharmacogenetic models of alcoholism. Alcoholism: Clin. Exp. Res. 3: 182-185, 1995.
5. McBride W.J., Murphy J.M, Lumeng L. and Li T.K. Serotonin, dopamine and GABA involvement in alcohol drinking of selectively bred rats. Alcohol 7: 199-205, 1990.
6. Murphy J.M., McBride W.J. Lumeng L. and Li T.K. Effects of serotonin and dopamine agents on ethanol intake of alcohol-preferring P rats. Alcoholism: Clin. Exp. Res. 12: 306, 1988.
7. Overstreet D.H. Kampov-Polevoy A.B. Rezvani A.H. Braun C. Bartus R.T. and Crews FT. Suppression of alcohol intake by chronic naloxone treatment in P rats: tolerance development and elevation of opiate receptor binding. Alcohol: Clin. Exp. Res. 23: 1761-1771, 1999.
8. Overstreet, D.H:, Rezvani, A.H., Janowsky, D.S. Genetic animal models of depression and ethanol preference provide support for cholinergic and serotonergic involvement in depression and alcoholism. Biol. Psychiat 31:919-936, 1992.
9. Overstreet D,H, Keung W.M., Rezvani A.H., Massi M., Lee D.Y. Herbal remedies for alcoholism: Promises and possible pitfalls. Alcol. Clin. Exp. Res. 27:177-185, 2003.
10. Rezvani, A.H., D.H. Overstreet and D.S. Janowsky. Genetic serotonin deficiency and alcohol preference in the fawn hooded rats. Alcohol and Alcoholism, 25:573-575, 1990.
11. Rezvani, A.H., D.H. Overstreet and D.S. Janowsky. Drug-induced reductions in ethanol intake in alcohol preferring and Fawn-Hooded rats. Alcohol and Alcoholism, Suppl. 1:433-437, 1991.
12. Rezvani, A.H., Garbutt, J.C., Shimoda, K., Garges, P.L., Janowsky, D.S., Mason, G.A. Attenuation of alcohol preference in alcohol preferring rats by a novel TRH analogue TA-0910. Alcoholism Clin Exp Res 16:326-330, 1992a.
13. Rezvani, A.H., Garges, P.L., Miller, D. and Christopher J. Gordon. Attenuation of alcohol consumption by MDMA in two strains of alcohol drinking rats. Pharmacol Biochem Behav 43:103-107, 1992b.
14. Rezvani, A.H., Grady, D.R. and Olgierd Pucilowski. Nimodipine, a Ca2+ channel antagonist, attenuates alcohol preference in alcohol preferring rats. Drugs in Development 2:143-151, 1993.
15. Rezvani, A.H. and Grady, D.R. Suppression of alcohol consumption by fenfluramine in Fawn-Hooded rats with serotonin dysfunction. Pharmacol Biochem Behav 48:105-110, 1994.
16. Rezvani, A.H., Peek, A.E., Grady, D.R. and O. Pucilowski. Inhibition of nitric oxide (NO) synthesis attenuated alcohol consumption in two strains of alcohol preferring rats. Pharmacol Biochem Behav 50:265-270, 1995.
17. Rezvani A.H., Overstreet D.H., Ying Y. and Clark Jr. E. Attenuation of alcohol intake by the extract of hypericum perforatum (St. John's Wort) in two strains of alcohol preferring rats. Alcohol & . Alcoholism 34:699-705, 1999.
18. Rezvani A.H. Overstreet D.H., Mason G.A., Janowsky D.S., Hamedi M., Clark Jr. and Ying Y. Combination pharmacotherapy: A mixture of small doses of naltrexone, fluoxetine, and a TRH analog reduces alcohol intake in three strains of alcohol preferring rats. Alcohol & Alcoholism: 35:76-83, 2000.
19. Rezvani A.H., Overstreet D.H., Perfumi M, Massi M. Plant derivatives in the treatment of alcohol dependency. Pharmacol. Biochem. Behav. 75:593-606, 2003.

## Claims

1. A carbamate compound or enantiomer selected from: 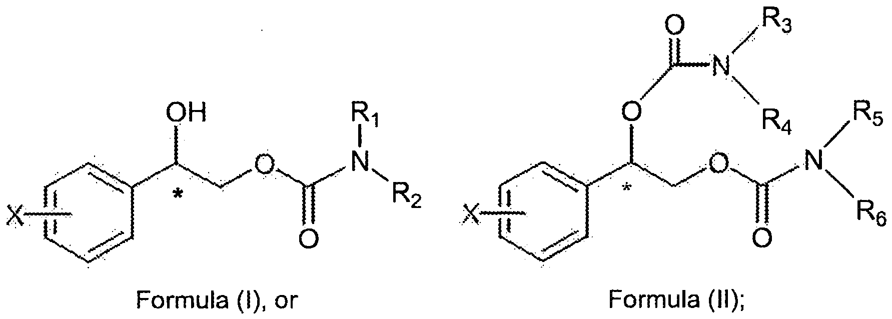 or an enantiomer selected from Formula (I) or Formula (II) in an enantiomeric mixture wherein one enantiomer predominates, or pharmaceutically acceptable crystalline or polymorphic forms thereof or a pharmaceutically acceptable salt thereof for use in preventing, treating or ameliorating substance-related disorders selected from drug withdrawal disorders, such as alcohol withdrawal with or without perceptual disturbances and/or delirium, amphetamine withdrawal, cocaine withdrawal, nicotine withdrawal, opioid withdrawal, sedative withdrawal, hypnotic withdrawal, anxiolytic withdrawal with or without perceptual disturbances and/or delirium, nicotine withdrawal with or without disturbances associated with smoking cessation and withdrawal symptoms due to other substances, substance-induced anxiety disorder with onset during withdrawal; vulnerability to environmental trigger induced craving; substance-induced mood disorder with onset during withdrawal; and substance-induced sleep disorder with onset during withdrawal, wherein
phenyl is substituted at X with one to five halogen atoms selected from the group consisting of fluorine, chlorine, bromine and iodine; and,
R₁, R₂, R₃, R₄, R₅ and R₆ are independently selected from the group consisting of hydrogen and C₁-C₄ alkyl; wherein C₁-C₄ alkyl is optionally substituted with phenyl and, wherein phenyl is optionally substituted with substituents independently selected from the group consisting of halogen, C₁-C₄ alkyl, C₁-C₄ alkoxy, amino, nitro and cyano.

2. The enantiomer of claim 1, wherein the enantiomer selected from Formula (I) or Formula (II) is an enantiomer selected from Formula (Ia) or Formula (IIa) or an enantiomer selected from Formula (Ia) or Formula (IIa) in an enantiomeric mixture wherein one enantiomer predominates: 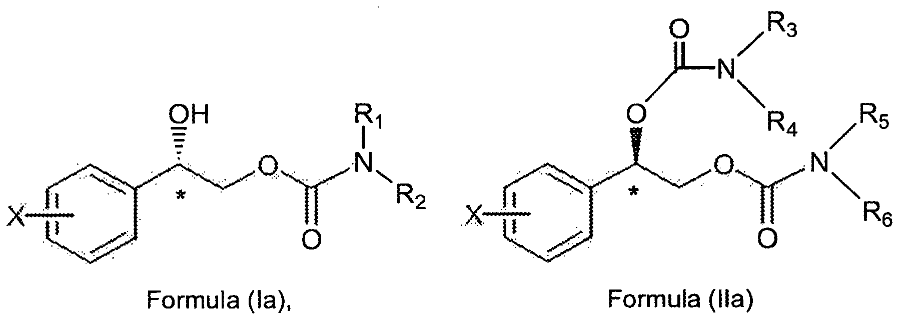 or pharmaceutically acceptable crystalline or polymorphic forms thereof or a pharmaceutically acceptable salt thereof, wherein
phenyl is substituted at X with one to five halogen atoms selected from the group consisting of fluorine, chlorine, bromine and iodine; and,
R₁, R₂, R₃, R₄, R₅ and R₆ are independently selected from the group consisting of hydrogen and C₁-C₄ alkyl; wherein C₁-C₄ alkyl is optionally substituted with phenyl (and, wherein phenyl is optionally substituted with substituents independently selected from the group consisting of halogen, C₁-C₄ alkyl, C₁-C₄ alkoxy, amino, nitro and cyano).

3. The carbamate compound of claim 1 or the enantiomer of claim 1 or 2, wherein X is chlorine.

4. The carbamate compound of claim 1 or the enantiomer of claim 1 or 2, wherein X is substituted at the ortho position of the phenyl ring.

5. The carbamate compound of claim 1 or the enantiomer of claim 1 or 2, wherein R₁, R₂, R₃, R₄, R₅ and R₆ are hydrogen.

6. The carbamate compound of claim 1, wherein the compound of Formula (I) is selected from an enantiomer of Formula (I), or an enantiomer of Formula (I) in an enantiomeric mixture wherein one enantiomer predominates.

7. The carbamate compound of claim 6, or the enantiomer of claim 1, wherein the enantiomer of Formula (I) in an enantiomeric mixture predominates to the extent of about 90% or greater.

8. The carbamate compound or enantiomer of claim 7, wherein the enantiomer of Formula (I) in an enantiomeric mixture predominates to the extent of about 98% or greater.

9. The carbamate compound of claim 1 wherein the compound of Formula (II) is selected from an enantiomer of Formula (II), or an enantiomer of Formula (II) in an enantiomeric mixture wherein one enantiomer predominates.

10. The carbamate compound of claim 9 or the enantiomer of claim 1, wherein the enantiomer of Formula (II) in an enantiomeric mixture predominates to the extent of about 90% or greater.

11. The carbamate compound or enantiomer of claim 10, wherein the enantiomer of Formula (II) in an enantiomeric mixture predominates to the extent of about 98% or greater.

12. The enantiomer of claim 1, wherein the enantiomer selected from Formula (I) or Formula (II) is an enantiomer selected from Formula (Ib) or Formula (IIb) or an enantiomer selected from Formula (Ib) or Formula (IIb) in an enantiomeric mixture wherein one enantiomer predominates: 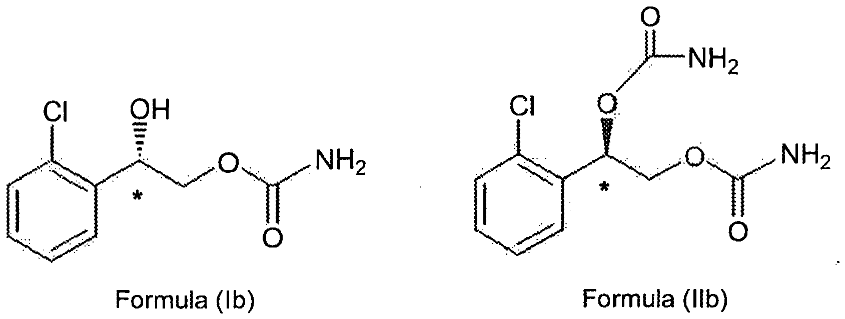 or pharmaceutically acceptable crystalline or polymorphic forms thereof or a pharmaceutically acceptable salt thereof.

13. The carbamate compound of claim 1 or the enantiomer of any of claims 1, 2 and 12, wherein the carbamate compound or enantiomer further comprises co-administrating a combination product, wherein the combination product is selected from a compound of claim 1 or an enantiomer of any of claims 1, 2 and 12, or a pharmaceutical composition comprising a pharmaceutically acceptable carrier and a compound of claim 1 or an enantiomer of any of claims 1, 2 and 12 and one or more compounds known to treat substance-induced disorders.

14. The carbamate compound or the enantiomer of claim 13, wherein the compound known to treat substance-induced disorders is one or more compounds selected from the group consisting of; naltrexone, naloxone or other opioid receptor antagonists, fluoxetine or other SSRI antidepressants, thyrotropin-releasing hormone analogues, venlafaxine or other SNRI antidepressants, MAO Inhibitor antidepressants, tricyclic antidepressants, bupropion, lithium carbonate, anticonvulsants, acamprosate, disulfiram (antabuse), calcium channel blockers, serotonin antagonists, GABA-altering drugs, dopaminergic drugs, and N-methyl-D-aspartic acid (NMDA) glutamate receptor modulators.

15. The carbamate compound or the enantiomer of claim 13, wherein the compound known to treat substance-induced disorders is one or more compounds selected from the group consisting of acamprosate, naltrexone, naloxone and disulfiram.

16. The carbamate compound of claim 1 or the enantiomer of any of claims 1, 2 and 12, wherein said carbamate compound or enantiomer is administered in an amount of from 0.01 µg/Kg/dose to 300 mg/Kg/dose.

17. The enantiomer of claim 2, wherein the enantiomer of Formula (Ia) in an enantiomeric mixture predominates to the extent of about 90% or greater.

18. The enantiomer of claim 17, wherein the enantiomer of Formula (Ia) in an enantiomeric mixture predominates to the extent of about 98% or greater.

19. The enantiomer of claim 2, wherein the enantiomer of Formula (IIa) in an enantiomeric mixture predominates to the extent of about 90% or greater.

20. The enantiomer of claim 19, wherein the enantiomer of Formula (IIa) in an enantiomeric mixture predominates to the extent of about 98% or greater.

21. The enantiomer of claim 12, wherein the enantiomer of Formula (Ib) in an enantiomeric mixture predominates to the extent of about 90% or greater.

22. The enantiomer of claim 21, wherein the enantiomer of Formula (Ib) in an enantiomeric mixture predominates to the extent of about 98% or greater.

23. The enantiomer of claim 12, wherein the enantiomer of Formula (IIb) in an enantiomeric mixture predominates to the extent of about 90% or greater.

24. The enantiomer of claim 23, wherein the enantiomer of Formula (IIb) in an enantiomeric mixture predominates to the extent of about 98% or greater.

## Patentansprüche

1. Carbamatverbindung oder Enantiomer ausgewählt aus: 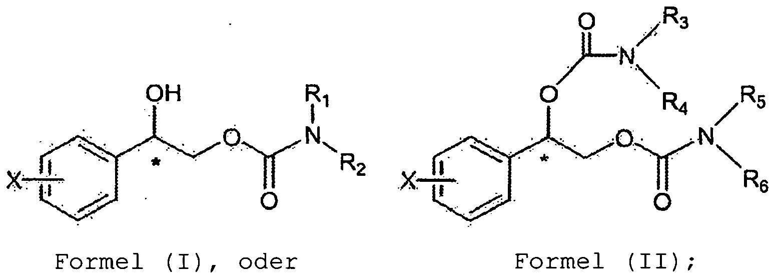 oder Enantiomer ausgewählt aus Formel (I) oder Formel (II) in einer Enantiomerenmischung, in welcher ein Enantiomer vorwiegt, oder pharmazeutisch unbedenkliche kristalline oder polymorphe Formen davon oder pharmazeutisch unbedenkliches Salz davon zur Verwendung bei der Prävention, Behandlung oder Linderung von mit Substanzen in Zusammenhang stehenden Erkrankungen ausgewählt aus Drogenentzugserkrankungen wie Alkoholentzug mit oder ohne Wahrnehmungsstörungen und/oder Delirium, Amphetaminentzug, Kokainentzug, Nikotinentzug, Opioidentzug, Beruhigungsmittelentzug, Hypnotikumentzug, Anxiolytikumentzug mit oder ohne Wahrnehmungsstörungen und/oder Delirium, Nikotinentzug mit oder ohne mit der Raucherentwöhnung assoziierten Störungen und Entzugssymptomen aufgrund von anderen Substanzen, während des Entzugs einsetzender substanzinduzierter Angststörungen, Anfälligkeit gegenüber durch die Umgebung ausgelöste Sucht, während des Entzugs einsetzender substanzinduzierter Gemütsstörungen und während des Entzugs einsetzender substanzinduzierter Schlafstörung, wobei
Phenyl an X durch ein bis fünf Halogenatome ausgewählt aus der aus Fluor, Chlor, Brom und Iod bestehenden Gruppe substituiert ist und
R₁, R₂, R₃, R₄, R₅ und R₆ unabhängig voneinander aus der aus Wasserstoff und C₁-C₄-Alkyl bestehenden Gruppe ausgewählt sind, wobei C₁-C₄-Alkyl gegebenenfalls durch Phenyl substituiert ist und wobei Phenyl gegebenenfalls durch unabhängig voneinander aus der aus Halogen, C₁-C₄-Alkyl, C₁-C₄-Alkoxy, Amino, Nitro und Cyano ausgewählte Substituenten substituiert ist.

2. Enantiomer nach Anspruch 1, wobei es sich bei dem aus Formel (I) oder Formel (II) ausgewählten Enantiomer um ein Enantiomer ausgewählt aus Formel (Ia) oder Formel (IIa) oder ein Enantiomer ausgewählt aus Formel (Ia) oder Formel (IIa) in einer Enantiomerenmischung, in welcher ein Enantiomer vorwiegt, handelt: 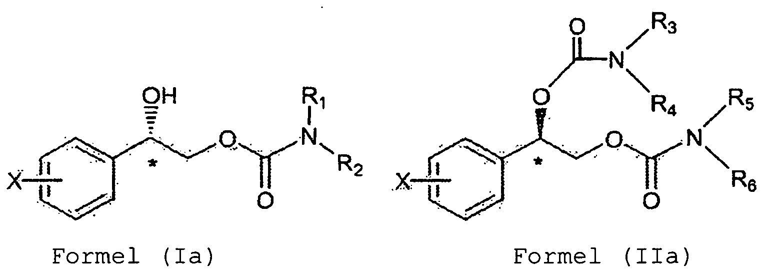 oder pharmazeutisch unbedenkliche kristalline oder polymorphe Formen davon oder ein pharmazeutisch unbedenkliches Salz davon, wobei
Phenyl an X durch ein bis fünf Halogenatome ausgewählt aus der aus Fluor, Chlor, Brom und Iod bestehenden Gruppe substituiert ist und
R₁, R₂, R₃, R₄, R₅ und R₆ unabhängig voneinander aus der aus Wasserstoff und C₁-C₄-Alkyl bestehenden Gruppe ausgewählt sind, wobei C₁-C₄-Alkyl gegebenenfalls durch Phenyl substituiert ist (und wobei Phenyl gegebenenfalls durch unabhängig voneinander aus der aus Halogen, C₁-C₄-Alkyl, C₁-C₄-Alkoxy, Amino, Nitro und Cyano bestehenden Gruppe ausgewählte Substituenten substituiert ist).

3. Carbamatverbindung nach Anspruch 1 oder Enantiomer nach Anspruch 1 oder 2, wobei X für Chlor steht.

4. Carbamatverbindung nach Anspruch 1 oder Enantiomer nach Anspruch 1 oder 2, wobei X an der Ortho-Stellung des Phenylrings substituiert ist.

5. Carbamatverbindung nach Anspruch 1 oder Enantiomer nach Anspruch 1 oder 2, wobei R₁, R₂, R₃, R₄, R₅ und R₆ für Wasserstoff steht.

6. Carbamatverbindung nach Anspruch 1, wobei die Verbindung der Formel (I) aus einem Enantiomer der Formel (I) oder einem Enantiomer der Formel (I) in einer Enantiomerenmischung, in welcher ein Enantiomer vorwiegt, ausgewählt ist.

7. Carbamatverbindung nach Anspruch 6 oder Enantiomer nach Anspruch 1, wobei das Enantiomer der Formel (I) in einer Enantiomerenmischung in einem Maße von etwa 90% oder mehr vorwiegt.

8. Carbamatverbindung oder Enantiomer nach Anspruch 7, wobei das Enantiomer der Formel (I) in einer Enantiomerenmischung in einem Maße von etwa 98% oder mehr vorwiegt.

9. Carbamatverbindung nach Anspruch 1, wobei die Verbindung der Formel (II) aus einem Enantiomer der Formel (II) oder einem Enantiomer der Formel (II) in einer Enantiomerenmischung, in welcher ein Enantiomer vorwiegt, ausgewählt ist.

10. Carbamatverbindung nach Anspruch 9 oder Enantiomer nach Anspruch 1, wobei das Enantiomer der Formel (II) in einer Enantiomerenmischung in einem Maße von etwa 90% oder mehr vorwiegt.

11. Carbamatverbindung oder Enantiomer nach Anspruch 10, wobei das Enantiomer der Formel (II) in einer Enantiomerenmischung in einem Maße von etwa 98% oder mehr vorwiegt.

12. Enantiomer nach Anspruch 1, wobei es sich bei dem aus Formel (I) oder Formel (II) ausgewählten Enantiomer um ein Enantiomer ausgewählt aus Formel (Ib) oder Formel (IIb) oder ein Enantiomer ausgewählt aus Formel (Ib) oder Formel (IIb) in einer Enantiomerenmischung, in welcher ein Enantiomer vorwiegt, handelt: 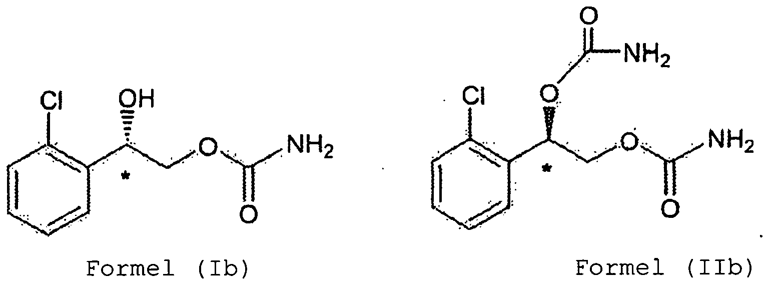 oder pharmazeutisch unbedenkliche kristalline oder polymorphe Formen davon oder ein pharmazeutisch unbedenkliches Salz davon.

13. Carbamatverbindung nach Anspruch 1 oder Enantiomer nach einem der Ansprüche 1, 2 und 12, wobei die Carbamatverbindung bzw. das Enantiomer weiterhin die gemeinsame Verabreichung eines Kombinationsprodukts umfasst, wobei das Kombinationsprodukt aus einer Verbindung nach Anspruch 1 oder einem Enantiomer nach einem der Ansprüche 1, 2 und 12 oder einer einen pharmazeutisch unbedenklichen Träger und eine Verbindung nach Anspruch 1 oder ein Enantiomer nach einem der Ansprüche 1, 2 und 12 und eine oder mehrere zur Behandlung von durch Substanzen induzierten Erkrankungen bekannte Verbindungen enthaltenden pharmazeutischen Zusammensetzung ausgewählt ist.

14. Carbamatverbindung oder Enantiomer nach Anspruch 13, wobei es sich bei der zur Behandlung von durch Substanzen induzierten Erkrankungen bekannten Verbindung um eine oder mehrere Verbindungen ausgewählt aus der aus Naltrexon, Naloxon oder anderen Opioidrezeptorantagonisten, Fluoxetin oder anderen SSRI-Antidepressiva, Analoga des thyrotropinfreisetzenden Hormons, Venlafaxin oder anderen SNRI-Antidepressiva, MAO-Inhibitor-Antidepressiva, tricyclischen Antidepressiva, Bupropion, Lithiumcarbonat, Antikonvulsiva, Acamprosat, Disulfiram (Antabuse), Calciumkanalblockern, Serotoninantagonisten, GABA-verändernden Arzneimitteln, dopaminergen Arzneimitteln und Modulatoren des N-Methyl-D-Asparaginsäure-(NMDA-)Glutamat-Rezeptors bestehenden Gruppe handelt.

15. Carbamatverbindung oder Enantiomer nach Anspruch 13, wobei es sich bei der zur Behandlung von durch Substanzen induzierten Erkrankungen bekannten Verbindung um eine oder mehrere Verbindungen ausgewählt aus der aus Acamprosat, Naltrexon, Naloxon und Disulfiram bestehenden Gruppe handelt.

16. Carbamatverbindung nach Anspruch 1 oder Enantiomer nach einem der Ansprüche 1, 2 und 12, wobei die Carbamatverbindung bzw. das Enantiomer in einer Menge von 0,01 µg/kg/Dosis bis 300 mg/kg/Dosis verabreicht wird.

17. Enantiomer nach Anspruch 2, wobei das Enantiomer der Formel (Ia) in einer Enantiomerenmischung in einem Maß von etwa 90% oder mehr vorwiegt.

18. Enantiomer nach Anspruch 17, wobei das Enantiomer der Formel (Ia) in einer Enantiomerenmischung in einem Maß von etwa 98% oder mehr vorwiegt.

19. Enantiomer nach Anspruch 2, wobei das Enantiomer der Formel (IIa) in einer Enantiomerenmischung in einem Maß von etwa 90% oder mehr vorwiegt.

20. Enantiomer nach Anspruch 19, wobei das Enantiomer der Formel (IIa) in einer Enantiomerenmischung in einem Maß von etwa 98% oder mehr vorwiegt.

21. Enantiomer nach Anspruch 12, wobei das Enantiomer der Formel (Ib) in einer Enantiomerenmischung in einem Maß von etwa 90% oder mehr vorwiegt.

22. Enantiomer nach Anspruch 21, wobei das Enantiomer der Formel (Ib) in einer Enantiomerenmischung in einem Maß von etwa 98% oder mehr vorwiegt.

23. Enantiomer nach Anspruch 12, wobei das Enantiomer der Formel (IIb) in einer Enantiomerenmischung in einem Maß von etwa 90% oder mehr vorwiegt.

24. Enantiomer nach Anspruch 23, wobei das Enantiomer der Formel (IIb) in einer Enantiomerenmischung in einem Maß von etwa 98% oder mehr vorwiegt.

## Revendications

1. Composé du carbamate ou énantiomère choisi parmi : 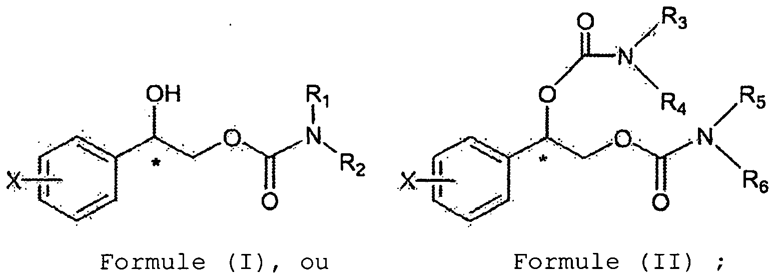 ou un énantiomère choisi parmi la formule (I) ou la formule (II) dans un mélange d'énantiomères dans lequel un énantiomère est majoritaire, ou des formes cristallines ou polymorphes pharmaceutiquement acceptables de celui-ci ou un sel pharmaceutiquement acceptable de celui-ci, destiné à être utiliser pour prévenir, traiter ou améliorer des troubles liés à des substances choisis parmi les troubles de sevrage de drogue, tels que le sevrage d'alcool avec ou sans perturbations de la perception et/ou délire, le sevrage d'amphétamine, le sevrage de cocaïne, le sevrage de nicotine, le sevrage d'opioïde, le sevrage de sédatif, le sevrage d'hypnotique, le sevrage d'anxiolytique avec ou sans perturbations de la perception et/ou délire, le sevrage de nicotine avec ou sans perturbations associées au sevrage tabagique et les symptômes dus à d'autres substances, le trouble d'anxiété induit par des substances avec démarrage pendant le sevrage ; la vulnérabilité à l'appétence induite par un facteur environnemental ; le trouble de l'humeur induit par des substances avec démarrage pendant le sevrage ; et le trouble du sommeil induit par des substances avec démarrage pendant le sevrage, où
phényle est substitué au niveau de X par un à cinq atomes d'halogène choisis dans le groupe constitué de fluore, de chlore, de brome et d'iode ; et
R₁, R₂, R₃, R₄, R₅ et R₆ sont choisis indépendamment dans le groupe constitué d'hydrogène et d'alkyle en C₁-C₄ ; où alkyle en C₁-C₄ est éventuellement substitué par phényle et où phényle est éventuellement substitué par des substituants choisis indépendamment dans le groupe constitué d'halogène, alkyle en C₁-C₄, alcoxy en C₁-C₄, amino, nitro et cyano.

2. Enantiomère de la revendication 1, **caractérisé en ce que** l'énantiomère choisi parmi la formule (I) ou la formule (II) est un énantiomère choisi parmi la formule (Ia) ou la formule (IIa) ou un énantiomère choisi parmi la formule (Ia) ou la formule (IIa) dans un mélange d'énantiomères dans lequel un énantiomère est majoritaire : 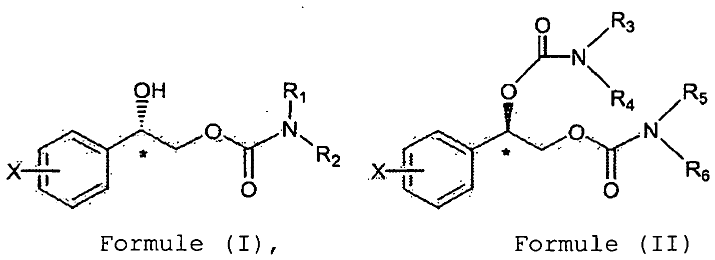 ou les formes cristallines ou polymorphes pharmaceutiquement acceptables de celui-ci ou un sel pharmaceutiquement acceptable de celui-ci, où
phényle est substitué au niveau de X par un à cinq atomes d'halogène choisis dans le groupe constitué de fluore, de chlore, de brome et d'iode ; et
R₁, R₂, R₃, R₄, R₅ et R₆ sont choisis indépendamment dans le groupe constitué d'hydrogène et d'alkyle en C₁-C₄ ; où alkyle en C₁-C₄ est éventuellement substitué par phényle (et où phényle est éventuellement substitué par des substituants choisis indépendamment dans le groupe constitué d'halogène, alkyle en C₁-C₄, alcoxy en C₁-C₄, amino, nitro et cyano).

3. Composé du carbamate de la revendication 1 ou énantiomère de la revendication 1 ou 2, **caractérisé en ce que** X est le chlore.

4. Composé du carbamate de la revendication 1 ou énantiomère de la revendication 1 ou 2, **caractérisé en ce que** X est substitué en position ortho du cycle phényle.

5. Composé du carbamate de la revendication 1 ou énantiomère de la revendication 1 ou 2, **caractérisé en ce que** R₁, R₂, R₃, R₄, R₅ et R₆ sont hydrogène.

6. Composé du carbamate de la revendication 1, **caractérisé en ce que** le composé de formule (I) est choisi parmi un énantiomère de formule (I), ou un énantiomère de formule (I) dans un mélange d'énantiomères dans lequel un énantiomère est majoritaire.

7. Composé du carbamate de la revendication 6, ou énantiomère de la revendication 1, **caractérisé en ce que** l'énantiomère de formule (I) dans un mélange d'énantiomères est majoritaire à hauteur d'environ 90 % ou plus.

8. Composé du carbamate ou énantiomère de la revendication 7, **caractérisé en ce que** l'énantiomère de formule (I) dans un mélange d'énantiomères est majoritaire à hauteur d'environ 98 % ou plus.

9. Composé du carbamate de la revendication 1, **caractérisé en ce que** le composé de formule (II) est choisi parmi un énantiomère de formule (II), ou un énantiomère de formule (II) dans un mélange d'énantiomères dans lequel un énantiomère est majoritaire.

10. Composé du carbamate de la revendication 9 ou l'énantiomère de la revendication 1, **caractérisé en ce que** l'énantiomère de formule (II) dans un mélange d'énantiomères est majoritaire à hauteur d'environ 90 % ou plus.

11. Composé du carbamate ou énantiomère de la revendication 10, **caractérisé en ce que** l'énantiomère de formule (II) dans un mélange d'énantiomères est majoritaire à hauteur d'environ 98 % ou plus.

12. Enantiomère de la revendication 1, **caractérisé en ce que** l'énantiomère choisi parmi la formule (I) ou la formule (II) est un énantiomère choisi parmi la formule (Ib) ou la formule (IIb) ou un énantiomère choisi parmi la formule (Ib) ou la formule (IIb) dans un mélange d'énantiomères dans lequel un énantiomère est majoritaire : 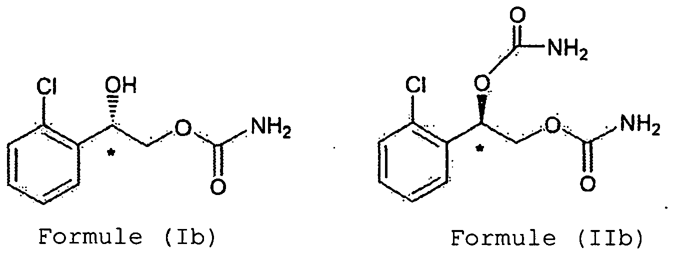 ou les formes cristallines ou polymorphes pharmaceutiquement acceptables de celui-ci ou un sel pharmaceutiquement acceptable de celui-ci.

13. Composé du carbamate de la revendication 1 ou énantiomère de l'une quelconque des revendications 1, 2 et 12, **caractérisé en ce que** le composé du carbamate ou l'énantiomère comprend en outre la co-administration d'un produit combiné, où le produit combiné est choisi parmi un composé de la revendication 1 ou un énantiomère de l'une quelconque des revendications 1, 2 et 12, ou une composition pharmaceutique comprenant un support pharmaceutiquement acceptable et un composé de la revendication 1 ou un énantiomère de l'une quelconque des revendications 1, 2 et 12 et un ou plusieurs composés connus pour traiter des troubles induits par des substances.

14. Composé du carbamate ou l'énantiomère de la revendication 13, **caractérisé en ce que** le composé connu pour traiter des troubles induits par des substances est un ou plusieurs composés choisis dans le groupe constitué de : naltrexone, naloxone ou d'autres antagonistes des récepteurs d'opioïdes, fluoxétine ou d'autres antidépresseurs de type SSRI, analogues de l'hormone de libération de la thyrotropine, venlafaxine ou d'autres antidépresseurs de type SNRI, antidépresseurs de type inhibiteurs de MAO, antidépresseurs tricycliques, bupropione, carbamate de lithium, anticonvulsivants, acamproaste, disulfirame (antabuse), bloqueurs de canaux calciques, antagonistes de la sérotonine, médicaments modificateurs de GABA, médicaments dopaminergiques, et modulateurs des récepteurs du glutamate acide N-méthyl-D-aspartique (NMDA).

15. Composé du carbamate ou énantiomère de la revendication 13, **caractérisé en ce que** le composé connu pour traiter des troubles induits par des substances est un ou plusieurs composés choisis dans le groupe constitué d'acamprosate, de naltrexone, de naloxone et de disulfirame.

16. Composé du carbamate de la revendication 1 ou énantiomère de l'une quelconque des revendications 1, 2 et 12, **caractérisé en ce que** ledit composé du carbamate ou énantiomère est administré dans une quantité de 0,01 µg/kg/dose à 300 mg/kg/dose.

17. Enantiomère de la revendication 2, **caractérisé en ce que** l'énantiomère de formule (Ia) dans un mélange d'énantiomères est majoritaire à hauteur d'environ 90 % ou plus.

18. Enantiomère de la revendication 17, **caractérisé en ce que** l'énantiomère de formule (Ia) dans un mélange d'énantiomères est majoritaire à hauteur d'environ 98 % ou plus.

19. Enantiomère de la revendication 2, **caractérisé en ce que** l'énantiomère de formule (IIa) dans un mélange d'énantiomères est majoritaire à hauteur d'environ 90 % ou plus.

20. Enantiomère de la revendication 19, **caractérisé en ce que** l'énantiomère de formule (IIa) dans un mélange d'énantiomères est majoritaire à hauteur d'environ 98 % ou plus.

21. Enantiomère de la revendication 12, **caractérisé en ce que** l'énantiomère de formule (Ib) dans un mélange d'énantiomères est majoritaire à hauteur d'environ 90 % ou plus.

22. Enantiomère de la revendication 21, **caractérisé en ce que** l'énantiomère de formule (Ib) dans un mélange d'énantiomères est majoritaire à hauteur d'environ 98 % ou plus.

23. Enantiomère de la revendication 12, **caractérisé en ce que** l'énantiomère de formule (IIb) dans un mélange d'énantiomères est majoritaire à hauteur d'environ 90 % ou plus.

24. Enantiomère de la revendication 23, **caractérisé en ce que** l'énantiomère de formule (IIb) dans un mélange d'énantiomères est majoritaire à hauteur d'environ 98 % ou plus.
